# EUROPEAN PATENT APPLICATION

(11) **EP 3 085 686 A1**
(43) Date of publication of application: **26.10.2016**
(21) Application number: 14872787.8
(22) Date of filing: 17.12.2014
(51) Int. Cl.: C07C 51/36, C07C 67/303, C07C 67/62, B01J 23/40

(54) **HYDROGENATION METHOD OF PHTHALATE COMPOUND**

(30) Priority: 19.12.2013 KR 20130159584; 03.09.2014 KR 20140116882
(71) Applicant: Hanwha Chemical Corporation, Seoul 04541 (KR)
(72) Inventor: JUNG, Ki Taeg, Daejeon 305-313 (KR); KIM, Hyo Suk, Daejeon 305-755 (KR); YOON, Kyong Jun, Daejeon 305-761 (KR); HAN, Kee Do, Daejeon 305-759 (KR); KWAK, Jang Young, Daejeon 305-804 (KR); KIM, Young Jo, Daejeon 305-790 (KR); SEONG, Pil Je, Daejeon 305-805 (KR); LEE, Sang Wook, Daejeon 305-720 (KR); ZHANG, Ying, Daejeon 305-805 (KR)
(74) Representative: Alt, Michael
(86) International application number: PCT/KR2014/012488
(87) International publication number: WO 2015/093849

(57) **Abstract**

There are provided a method for hydrogenation of a phthalate compound. According to the method for hydrogenation of the present invention, a hydrogenation reaction is performed in a multi-pipe type reactor in a state in which a viscosity of only a liquid-phase phthalate based raw material is lowered, such that a yield of a hydrogenation reaction process may be improved, and operation stability and economical efficiency on a commercial scale may be improved by hot spot control. According to another method of the present invention, long-term activity of a catalyst used in a reaction is maintained, and performance of the catalyst may be improved, such that stability and economical efficiency of a hydrogenation process may be improved. Therefore, a total amount of the catalyst required in the reaction may be decreased, and a replacement cycle may be extended, thereby making it possible to improving operation stability of the process and economical efficiency at the time of performing the process on a commercial scale.

## Description

### [Technical Field]

The present invention relates to a method for hydrogenation of a phthalate compound, and more particularly, to a method for hydrogenation of a phthalate compound capable of improving performance and life time of a catalyst used in the hydrogenation method.

### [Background]

A phthalate based compound is a widely used material as a plasticizer of plastics, particularly, poly vinyl chloride (PVC). For example, the phthalate based compound may be variously used in electric and electronic products, medicines, paint pigments, lubricants, binders, surfactants, adhesives, tiles, food containers, package materials, and the like.

However, as some of the phthalate based compounds have been known as a material causing environmental contamination and human endocrinal disruption problems, regulations on use of the phthalate based compound has been intensified around advanced countries such as Europe, U.S., or the like, as an effort to decrease a use of the phthalate based compound. Particularly, among phthalate based plasticizers, some products such as di(2-ethylhexyl) phthalate (DEHP), butyl benzyl phthalate (BBP), and di-n-butyl phthalate (DBP) are socially suspected as environmental hormones, that is, endocrine disruptors inhibiting or disrupting hormone actions in the human body, such that there is the trend toward regulation on these products.

Therefore, there have been made efforts to develop an eco-friendly plasticizer free from debates on the environmental hormones while having performance equal to that of the plasticizer according to the related art. As one of the efforts, there is a method of using a compound in which a benzene ring included in the phthalate based compound is hydrogenated.

As a hydrogenation reaction of an aromatic compound such as the benzene ring, a method using a catalyst in which a transition metal such as ruthenium is contained on a support as an active material has been known.

However, among the phthalate compound used in the catalytic reaction, compounds having a viscosity of 20 cP or more at room temperature (20°C) decrease permeability of hydrogen in forming a film layer on a catalyst surface in a fixed-bed catalytic reactor, such that the hydrogenation reaction of the compound is not smoothly carried out.

Further, in the case of increasing a reaction temperature at a predetermined level or more in order to decrease the viscosity of the compound, side reactions in the reactor are increased, such that a yield of raw materials may be deteriorated.

Therefore, in order to produce a material capable of being used as an eco-friendly plasticizer on an industrial scale, in the phthalate compound having a viscosity of 20 cP or more, reaction condition ranges at a predetermined level have been required.

### [Disclosure]

### [Technical Problem]

The present invention has been made in an effort to provide a method for hydrogenation of a phthalate compound capable of improving process operability and increasing economical efficiency of a commercial process by improving hydrogenation reaction performance of a phthalate based compound and increasing a reaction yield.

In addition, the present invention has been made in an effort to provide a method for hydrogenation of a phthalate compound capable of improving performance of a catalyst used in a hydrogenation reaction of a phthalate based compound and maintaining activity of the catalyst to extend a life time of the catalyst.

### [Technical Solution]

An exemplary embodiment of the present invention provides a method for hydrogenation of a phthalate compound including:
lowering a viscosity in a reactor to 10 cp or less with respect to a phthalate compound having a viscosity of 20 cP or more at room temperature, and then reacting hydrogen with the phthalate compound, in the presence of a hydrogenation catalyst in the reactor.

Another exemplary embodiment of the present invention provides a method for hydrogenation of a phthalate compound including: reacting the phthalate compound with hydrogen, in the presence of a hydrogenation catalyst and an alcohol having at least two carbon atoms.

### [Advantageous Effects]

With the hydrogenation method according to an exemplary embodiment of the present invention, hydrogenation reaction performance may be improved and the reaction yield may be improved in a predetermined range by adjusting reaction conditions at which the hydrogenation reaction is carried out in a fixed-bed catalytic reactor.

In addition, according to the method of the present invention, reaction conditions of a predetermined level are reached, and accordingly, a viscosity of the phthalate compound reached a predetermined level or less, such that a thin film may be formed on a catalyst surface, which may increase diffusion of the phthalate compound and wettability of a catalyst, thereby making it possible to improve efficiency of the catalyst. Further, permeability of a hydrogen raw material to the catalyst is increased, such that reaction efficiency may be further increased.

In addition, in the hydrogenation method according to the present invention, the generation of side reactions may be significantly decreased in the reaction, such that a high yield may be obtained in the reactor, thereby making it possible to improve economical efficiency at the time of operating a commercial process.

Furthermore, generation of a high temperature by reaction heat in the reactor in which the hydrogenation reaction is performed may be suppressed, stagnation of a flow of the liquid-phase may be suppressed by lowering a viscosity of the reaction raw material, and reactivity may be improved by forming a film on the catalyst surface to be thinner.

In addition, a preferable reactor of the present invention is composed of a multi-pipe tube in the reactor, such that the reactor is advantageous in view of controlling reaction heat generated in a gas-phase/liquid-phase reaction. In addition, since heat transfer performance in the tube may be improved due to a low viscosity of the liquid-phase raw material, the reactor may be advantageous in view of controlling a reaction temperature. Therefore, generation of a hot spot in the catalyst surface in the reactor may be suppressed, such that a life time of the catalyst and a reaction yield may be increased.

Therefore, a viscosity range of the liquid-phase raw material in the reactor is adjusted in a predetermined level or less, such that process productivity may be improved, a yield may be increased, and at the time of performing a process on a commercial scale, economical efficiency may be improved.

Further, according to the present invention, as an alcohol is additionally used at the time of hydrogenation reaction, long-term activity of the catalyst used in the reaction may be maintained and performance of the catalyst may be improved, thereby making it possible to improve stability and economical efficiency of a hydrogenation process. In addition, according to the present invention, a life time of the catalyst may be extended by suppressing a metal ion, a metal salt compound, or other impurity ingredients contained in the phthalate compound, which is a raw material, from being physically and chemically adsorbed in the catalyst. Therefore, a total amount of the catalyst required in the reaction may be decreased, and a replacement cycle may be extended, thereby making it possible to improving operation stability of the process and economical efficiency at the time of performing the process on a commercial scale.

### [Brief Description of the Drawings]

FIG. 1 is a view briefly showing a hydrogenation reaction process used in a hydrogenation method according to the present invention.
FIG. 2 is a view briefly showing a hydrogenation reaction apparatus having a continuous multi-pipe used in the hydrogenation method according to the present invention.
FIG. 3 is a view briefly showing a continuous circulation type hydrogenation reaction apparatus used in the hydrogenation method according to the present invention.
FIG. 4 is a view briefly showing a hydrogenation reaction apparatus used in a hydrogenation method according to the present invention.
FIGS. 5 and 6 show results obtained by performing nuclear magnetic resonance (NMR) analysis on an ingredient adsorbed in a catalyst surface after a hydrogenation reaction according to Comparative Example 3.

### [Best Mode]

The present invention may be variously modified and have various types, and specific embodiments of the present invention will be descried in detail. However, the present invention is not limited to the exemplary embodiments described herein, but all of the modifications, equivalents, and substitutions within the spirit and scope of the present invention are also included in the present invention. Further, when it is determined that the detailed description of the known art related to the present invention may obscure the gist of the present invention, the detailed description thereof will be omitted.

In addition, terms including an ordinal number such as first, second, or the like, to be used in the present specification may be used to describe various components. However, these components are not limited to these terms. The terms are only used to differentiate one component from other components. For example, the 'first' component may be named the 'second' component and the 'second' component may also be similarly named the 'first' component, without departing from the scope of the present invention.

Singular forms used in the specification are intended to include plural forms unless the context clearly indicates otherwise. Terms such as "include", "have", and the like, used in the present specification will imply the existence of stated features, numbers, steps, operations, configuration elements, components, or a combination thereof, but do not exclude other features, numbers, steps, operations, configuration elements, components, or a combination thereof.

Hereinafter, a hydrogenation method of a phthalate compound according to the present invention will be described in detail with reference to the accompanying drawings.

The present invention relates to a hydrogenation method of a phthalate compound capable of obtaining a high yield by a specific reaction at a predetermined temperature and pressure. Further, the present invention relates to a hydrogenation method capable of improving process operability and increasing economical efficiency by improving performance of a used catalyst and maintaining activity of the catalyst to extend a lift time of the catalyst.

That is, the method for hydrogenation of a phthalate compound according to the present invention includes a method of lowering a viscosity of a liquid-phase phthalate compound to a level of 10 cP or less and then performing a hydrogenation reaction or a method of lowing a liquid-phase mixture in a predetermined range by using an alcohol compound to perform the hydrogenation reaction. Therefore, according to the present invention, hydrogenation reactivity may be improved as compared to the related art, and process operability and economic efficiency may be improved by improving performance of the used catalyst and maintaining the activity of the catalyst to extend the life time of the catalyst.

According to a first exemplary embodiment of the present invention as described above, there is provided a method for hydrogenation of a phthalate compound including: lowering a viscosity in a reactor to a 10 cP or less with respect to a phthalate compound having a viscosity of 20 cP or more at room temperature, and then reacting hydrogen with the phthalate compound in the presence of a hydrogenation catalyst in the reactor.

In addition, the hydrogenation method according to the present invention may include: raising a pressure and temperature of a phthalate compound in order to lower a viscosity of the phthalate compound introduced into a reactor to 10 cP or less; supplying the phthalate compound having a viscosity of 10 cP or less and gas-phase hydrogen in the reactor filled with a catalyst; and reacting the phthalate compound having a viscosity of 10 cP or less with hydrogen.

In detail, the hydrogenation method according to the present invention includes: reacting the phthalate compound with hydrogen in the presence of the hydrogenation catalyst and reactor. Particularly, in the present invention, a yield of a hydrogenation reaction process may be improved by only the phthalate compound with gas-phase hydrogen in a state in which the viscosity of the phthalate compounding having a viscosity of 20 cP or more at room temperature (20 °C) is lowered to 10 cP or less instead of directly using the phthalate compound in the hydrogenation reaction, and operation stability and economical efficiency on a commercial scale may be improved through hot spot control.

In addition, the hydrogenation method according to the present invention may serve to increase a mass transfer effect with the catalyst instead of a concept of suppressing impurities adsorbed in the catalyst. That is, in the hydrogenation method according to the present invention, the viscosity of a liquid-phase raw material is lowered, and thus, generation of side reactions may be significantly decreased in the hydrogenation reaction, such that a high yield may be obtained in the reactor, thereby making it possible to improve economical efficiency at the time of operating a commercial process.

The phthalate compound may be used in the reaction after lowering the viscosity to 10 cP or less in the raising of the pressure and temperature using at least one heat exchanger. More preferably, the raising of the pressure and temperature may be performed using at least one heat exchanger at a pressure of 50 to 500 bar and a temperature of 50 to 500 °C. Further, the number of heat exchanger is not limited but may be changed depending on a heat exchange method for the liquid-phase raw material. As the heat exchange method in the present invention, a method using reaction heat recovered from a reaction product after the reaction through a multi-pipe cylindrical structure or a reactor jacket may be used, but the present invention is not limited thereto.

In addition, the reactor used in the present invention is not particularly limited as long as it may be used in the art to which the present invention pertains. For example, a continuous type multi-pipe type reactor may be used. The reactor may include a heat control device controlling heat generated during the reaction. Further, the reactor is not limited to a multi-pipe type reactor, but various type reactors such as a circulation type reactor, a batch type reactor, and the like, may be applied.

According to an exemplary embodiment of the present invention, the reactor used in the hydrogenation reaction of the present invention may be a cylindrical multi-pipe type reactor having a multi-pipe formed therein. In addition, the multi-pipe type reactor may be connected and installed with a supply line for injecting the phthalate compound (liquid phase) of which the viscosity is lowered and hydrogen (gas phase), and installed with a cooling water inflow line and discharge line for recovering reaction heat generated in the hydrogenation reaction.

Further, the multi-pipe type reactor may be connected to a gas-liquid separator for recovering a reaction mixture and a recovery system for circulating an unreacted material to recycle the unreacted material. According to the present invention, after the hydrogenation reaction of the phthalate compound, some of the reactants obtained from a reaction apparatus may be circulated to a pump through a lower portion of the reactor, reaction heat may be removed through the heat exchanger, the reactants except for the catalyst may be recovered in the recovery system, and then the residual reactants and the catalyst may be introduced again into the reactor.

As described above, a hydrogenation reaction apparatus according to the present invention is composed of a multi-pipe tube in the reactor, such that the hydrogenation reaction apparatus is advantageous in view of controlling reaction heat generated in a gas-phase/liquid-phase reaction. In addition, since heat transfer performance in the tube may be improved due to a low viscosity of the liquid-phase raw material, the hydrogenation reaction apparatus may be advantageous in view of controlling a reaction temperature. Therefore, generation of a hot spot in the catalyst surface in the reactor may be suppressed, such that the life time of the catalyst and the reaction yield may be increased.

Meanwhile, a reaction target of the hydrogenation method according to the present invention is the phthalate compound, and hydrogen is added to a benzene ring of the phthalate compound by the hydrogenation reaction to thereby be converted into a cyclohexane dicarboxylate compound corresponding to the phthalate compound.

The phthalate compound may be at least one selected from phthalate, terephthalate, isophthalate, and a carboxylic acid compound corresponding thereto.

First, the phthalate compound may be represented by the following Chemical Formula 1.

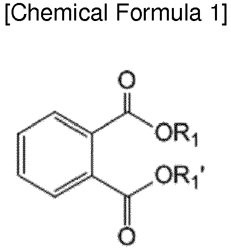

In Chemical Formula 1, R1 and R1' are each independently the same or different and are hydrogen, or a straight- or branched-chain alkyl group having 1 to 20 carbon atoms, preferably 4 to 20 carbon atoms, more preferably 5 to 20 carbon atoms, and most preferably, 5 to 10 carbon atoms.

A specific example of the phthalate compound may include dibutyl phthalate (DBP), dihexyl phthalate (DHP), dioctyl phthalate (DOP), di-n-octyl phthalate (DnOP), diisononyl phthalate, diisodecyl phthalate (DIDP), or the like, but is not limited thereto. One or a mixture of these compounds may be used.

The terephthalate compound may be represented by the following Chemical Formula 2.

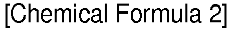

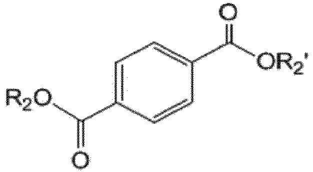

In Chemical Formula 2, R2 and R2' are each independently the same or different and are hydrogen, or a straight- or branched-chain alkyl group having 1 to 20 carbon atoms, preferably 4 to 20 carbon atoms, more preferably 5 to 20 carbon atoms, and most preferably, 5 to 10 carbon atoms.

A specific example of the terephthalate compound may include dibutyl terephthalate (DBTP), dioctyl terephthalate (DOTP), diisononyl terephthalate (DINTP), or diisodecyl terephthalate (DIDTP), but is not limited thereto. One or a mixture of these compounds may be used.

The isophthalate compound may be represented by the following Chemical Formula 3.

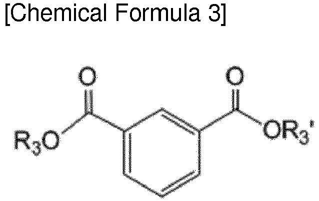

In Chemical Formula 3, R3 and R3' are each independently the same or different and are hydrogen, or a straight- or branched-chain alkyl group having 1 to 20 carbon atoms, preferably 4 to 20 carbon atoms, more preferably 5 to 20 carbon atoms, and most preferably, 5 to 10 carbon atoms.

A specific example of the isophthalate compound may include dibutyl isophthalate (DBIP), dioctyl isophthalate (DOIP), diisononyl isophthalate (DINIP), diisodecyl isophthalate (DIDIP), or the like, but is not limited thereto. One or a mixture of these compounds may be used.

It is preferable that dioctyl terephthalate (DOTP) is used as the phthalate compound.

A purity of the phthalate compound may be about 98% or more, preferably about 99%, and more preferably about 99.5% or more, but is not limited thereto. All of the phthalate compounds with quality and purity capable of being commercially used may be used.

In addition, as described above, according to the present invention, the viscosity is lowered by a series of processes in a liquid phase state before the phthalate compound is supplied to the reactor, such that a flow in the liquid phase state may be improved. Further, while the hydrogenation reaction of the phthalate compound proceeds after injecting the phthalate compound into the reactor, a uniform reaction may be entirely carried out due to a decrease in a film thickness of the liquid-phase raw material on each of the catalyst surfaces, and reaction heat in the reactor may be rapidly removed through a cooling medium due to a low viscosity of the phthalate compound. In addition, due to the above-mentioned effects, generation of the hot spot in the reactor may be suppressed, and the catalyst uniformly participates in the reaction, such that reaction efficiency may be improved, catalytic performance deterioration may be suppressed, and the reaction yield may be improved.

More specifically, the raising of the pressure and temperature of the phthalate compound may be simultaneously or sequentially performed, and the desired pressure and temperature may be reached by raising the pressure and temperature at one time or several times through a plurality of steps. For example, the phthalate compound may be injected into the reactor in a liquid phase state in which the phthalate compound has a suitable viscosity by raising the pressure of the phthalate compound and then raising a temperature of the pressure-raised phthalate compound through the heat exchanger.

According to an exemplary embodiment of the present invention, before injection into the reactor, the phthalate compound generally has a viscosity of 20 cP or more at room temperature. However, the raising of the pressure and temperature of the phthalate compound is performed according to the present invention, such that the viscosity of the phthalate compound at the time of injection into the reactor may be about 0.2 to 10.0 cP, more preferably about 2 to 5 cP in a pressure and temperature condition range when the phthalate compound is introduced into the reactor. When the viscosity of the phthalate compound is in the above-mentioned range, flowability may be excellent even after a predetermined time in the reactor, and reactivity with hydrogen may be increased.

At the time of raising the pressure for introduction into the reactor, the desired pressure may be about 50 to about 500 bars, preferably about 100 to about 300 bars. In the case in which the pressure is less than 50 bar, reactivity may be deteriorated, such that it may be difficult to obtain a conversion rate of the desired level, and in the case in which the pressure is more than 500 bar, the pressure is excessively high, such that it may be difficult to manufacture the reactor, or a manufacturing cost may be significantly increased.

In addition, at the time of raising the temperature for introduction into the reactor, the desired temperature may be about 50 to about 500 °C, preferably about 100 to about 300 °C. In the case in which the temperature is less than 50 °C, the catalyst becomes inactivated due to a low temperature, flowability in the reactor is deteriorated due to a high viscosity of the mixture, permeability of hydrogen to the phthalate compound in the liquid phase state is deteriorated, such that the reaction is not suitably performed in the reactor, and in the case in which the temperature is more than 500 °C, decomposition of the reactant may be increased, it may be difficult to manufacture the reactor, and it may be difficult to control heat, or the like, due to a rapid reaction.

In this case, pressure and temperature conditions of hydrogen may be adjusted so as to be equal to the pressure and temperature conditions of the phthalate compound, that is, to be in a pressure range of about 50 to about 500 bar, preferably about 100 to about 300 bar and a temperature range of about 50 to about 500 °C, preferably about 80 to about 300 °C.

The hydrogenation catalyst may contain a Group 8 transition metal as an active ingredient. That is, the hydrogenation catalyst may contain, preferably, at least one selected from ruthenium (Ru), nickel (Ni), palladium (Pd), rhodium (Rh), platinum (Pt), and the like.

An aromatic ring of the phthalate compound is hydrogenated by the hydrogenation reaction as described above to thereby be converted into the cyclohexane dicarboxylate compound corresponding thereto.

After the reaction is terminated, the produced liquid-phase hydrogenation reaction product and unreacted gas-phase raw material are separated from each other. The separated gas-phase raw material may be recirculated in the hydrogenation process. The recovered hydrogenation reaction product may be finally separated through a decompression and cooling process.

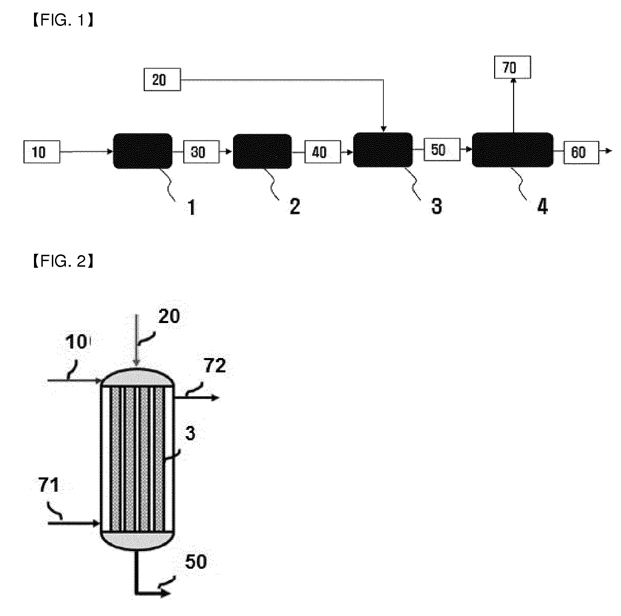

FIG. 1 is a view schematically showing a hydrogenation reaction apparatus used in the hydrogenation method according to the present invention.

Referring to FIG. 1, the hydrogenation reaction apparatus may be composed of a heat exchanger 1 heating a liquid-phase raw material, a heat exchanger 2 additionally heating the liquid-phase raw material, a multi-pipe type reactor 3, a gas-liquid separator 4, and the like. In addition, the number of heat exchanger heating the liquid-phase raw material is not limited. That is, at least one heat exchanger may be used, and the number of heat exchanger may be changed depending on the heat exchange method. As described above, the reactor 3 is not limited to the multi-pipe type reactor, but various type reactors such as the circulation type reactor, the batch type reactor, or the like, may be applied. Further, a recovery system reaction heat in the multi-pipe type reactor 3 may be removed by a separate heat exchanger outside the reactor, or the like.

Describing a process using the hydrogenation reaction apparatus according to an exemplary embodiment of the present invention in detail, first, after a phthalate compound 10 is primarily and secondarily heated through the heat exchangers 1 and 2, the heated phthalate compound is injected into the multi-pipe type reactor. In this case, the phthalate compound 10 having a high density becomes in a state of a primary phthalate compound 30 of which the viscosity is lowered and a secondary phthalate compound 40 having a viscosity of 0. 2 to 10 cP through the heat exchangers 1 and 2, and the secondary phthalate compound is injected into the reactor 3. Further, a hydrogen raw material 20 may be heated and then injected into the reactor 3 or be directly injected into the reactor without heating. The gas-phase and liquid-phase raw materials injected into the reactor 3 as described above react with each other in the presence of the catalyst, and recirculated hydrogen 70 and the reaction product 60 are separated in a mixture 50 in which the reaction product and residual hydrogen coexist after a final reaction through the gas-liquid separator 4.

In addition, FIG. 2 is a view briefly showing a multi-pipe type reactor used in the hydrogenation reaction according to an exemplary embodiment of the present invention.

Referring to FIG. 2, the liquid-phase raw material (phthalate compound) 10 having a lower viscosity and the gas-phase hydrogen 20 are introduced into the reactor 3 filled with the catalyst by raising the pressure and temperature, and a hydrogen reaction is performed on a multi-phase fluids over the catalyst. After reaction heat generated in this process is removed through inflow cooling water 71, discharge cooling water 72 is recovered. The recovered heat may be reused in other processes or removed through a cooling tower, but a treatment method of the removed reaction heat is not limited to the method as described above. In addition, the reaction mixture 50 obtained in the reactor 3 after the reaction is terminated may be obtained through a lower end of the reactor.

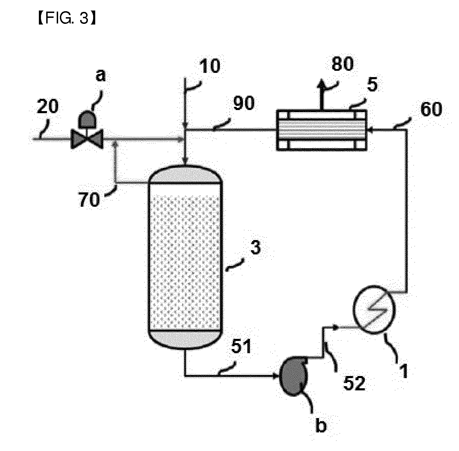

FIG. 3 is a view briefly showing a continuous circulation type hydrogenation reaction apparatus used in the hydrogenation method according to the present invention.

Referring to FIG. 3, after a predetermined amount of the hydrogen raw material 20 is supplied to a pressure value a and mixed with the recirculated hydrogen 70, the liquid-phase phthalate raw material 10 and a circulation material 90 in the reactor are all mixed and injected into the reactor 3, thereby carrying out the reaction. Some 51 of the reactant is circulated through a lower portion of the reactor and a pump b. In this case, reaction heat in the circulated material 52 is removed through the first heat exchanger 1. Thereafter, a reactant 80 except for the catalyst is recovered from the reaction heat-removed material 60 (including the reaction product) through a separator (reactant recovery system) 5, and the residual reactant and the catalyst are introduced into the reactor 3 again.

Meanwhile, according to another exemplary embodiment of the present invention, a hydrogenation method includes: reacting a phthalate compound and hydrogen in the presence of a hydrogenation catalyst and an alcohol having at least two carbon atoms.

A reaction target of the hydrogenation method according to the present invention is the phthalate compound, and hydrogen is added to a benzene ring of the phthalate compound by the hydrogenation reaction to thereby be converted into a cyclohexane dicarboxylate compound corresponding to the phthalate compound.

The phthalate compound mixed with the alcohol may be the same as that in the above-mentioned exemplary embodiment.

The phthalate compound as described above may be obtained by performing an esterification reaction of an acid such as phthalic acid, terephthalic acid, isophthalic acid or anhydride thereof with the alcohol. After the esterification reaction as described above, in order to neutralize a catalyst used in the esterification reaction and the residual acid ingredient, a basic compound such as sodium carbonate (Na₂CO₃), sodium hydroxide (NaOH), calcium carbonate (CaCO₃), and potassium hydroxide (KOH) is used. Therefore, a trace amount of impurities, for example, metal ions such as Na⁺, K⁺, and Ca²⁺ isolated from this basic compound, metal salt compounds formed by binding with the metal ions, or other reaction by-products, may remain in the phthalate compound.

The trace amount of the metal ion or metal salt compound contained in the phthalate compound does not have a large influence on quality of the phthalate compound, but acts as a catalyst poison on the hydrogenation catalyst used in the hydrogenation reaction of the phthalate compound, such that the metal ion or metal salt compound becomes one of the main causes of deterioration in activity of the catalyst. More specifically, the metal ion or metal salt compound is present in a state in which the metal ion or metal salt compound is not sufficiently dissolved or is dispersed, and the metal ion or metal salt compound may be easily physically or chemically adsorbed by the hydrogenation catalyst, such that an activity of the catalyst may be rapidly decreased.

However, according to the hydrogenation method of the present invention, the alcohol having at least two carbon atoms may serve to maintain the activity of the hydrogenation catalyst by effectively dissolving the metal ion, the metal salt compound, or other impurity ingredients to prevent adsorption by the hydrogenation catalyst.

As the alcohol, the alcohol having at least two carbon atoms, for example, one selected from ethanol, n-propanol, isopropanol, n-butanol, isobutanol, pentanol, hexanol, heptanol, n-octanol, 2-ethylhexanol, nonanol, decanol, undecanol, dodecanol, and the like, which are aliphatic alcohols having 2 to 12 carbon atoms, preferably, 2 to 10 carbon atoms, or a mixture thereof may be used.

A usable alcohol may be changed depending on the specific kind of phthalate compound, which is a reaction target. For example, in the case of performing the hydrogenation reaction on dioctyl terephthalate, among the alcohols, when an alcohol having 2 to 8 carbon atoms such as ethanol, butanol, or octanol is used, an effect of improving reactivity and extending the life time of the catalyst may be further increased.

According to the present invention, the alcohol lowers the viscosity of the mixture to improve a flow of the liquid-phase mixture in the liquid phase state before the alcohol is mixed with the phthalate compound to thereby be supplied to the reactor. Further, while the mixture is injected into the reactor and the hydrogenation reaction of the phthalate compound is performed, the alcohol serves to absorb reaction heat to suppress a high temperature from being generated by the reaction heat. In addition, the alcohol may improve reactivity of the catalyst by forming a thin film on a hydrogenation catalyst surface and suppress the metal ion, the metal salt compound, other impurity ingredients contained in the phthalate compound from being physically or chemically adsorbed by the catalyst to thereby extend the life time of the catalyst.

The alcohol may be contained at a content of about 5 to about 60 parts by weight, preferably, about 10 to about 50 parts by weight, more preferably, about 10 to 40 parts by weight, and most preferably about 10 to 30 parts by weight based on 100 parts by weight of the phthalate compound. In the case in which the content of the alcohol is less than 5 parts by weight, there is almost no effect of improving catalytic performance, and in the case in which the content is more than 60 parts by weight, there is a need to increase a size of the reactor and a large amount of energy is consumed in a separation process, such that economical efficiency may be deteriorated.

According to an exemplary embodiment of the present invention, the hydrogenation reaction may be performed by further mixing the cyclohexane dicarboxylate compound, which is the reaction product, in addition to the alcohol. In the case of further mixing the reaction product to perform the reaction, a violent reaction in the reactor may be suppressed, such that the reaction temperature may be controlled, and a phenomenon that catalytic performance is locally deteriorated over the catalyst may be decreased.

The hydrogenation process of the phthalate compound may be performed in the liquid phase or gas phase. According to an exemplary embodiment of the present invention, the hydrogenation reaction may be performed in a state in which the phthalate compound and alcohol are in a liquid state and hydrogen is in a gas state.

The hydrogenation method may further include, before injecting the phthalate compound and alcohol, mixing the phthalate compound and alcohol with each other so as to have a uniform concentration.

According to an exemplary embodiment of the present invention, the phthalate compound and alcohol are mixed with each other before being injected into the reactor, and after raising the pressure and temperature of the mixture including the phthalate compound and alcohol, the mixture may be injected into the reactor.

More specifically, the raising of the pressure and temperature of the mixture including the phthalate compound and alcohol may be simultaneously or sequentially performed, and the desired pressure and temperature may be reached by raising the pressure and temperature at one time or several times through a plurality of steps. For example, the mixture including the phthalate compound and alcohol may be injected into the reactor in a liquid phase state in which the mixture has a suitable viscosity by raising the pressure of the mixture and then raising a temperature of the pressure-raised mixture. According to an exemplary embodiment of the present invention, the viscosity of the mixture may be about 0.5 to about 20 cP in the pressure and temperature condition ranges when the mixture is injected into the reactor. Preferably, the viscosity of the liquid-phase mixture may be about 0.5 to 12 cP. When the viscosity of the mixture is in the above-mentioned range, suitable flowability and reactivity may be exhibited in the reactor.

At the time of raising the pressure for introduction into the reactor, the desired pressure may be about 50 to about 500 bars, preferably about 100 to about 300 bars. In the case in which the pressure is less than 50 bar, reactivity may be deteriorated, such that it may be difficult to obtain a conversion rate of the desired level, and in the case in which the pressure is more than 500 bar, the pressure is excessively high, such that it may be difficult to manufacture the reactor, or a manufacturing cost may be significantly increased.

In addition, at the time of raising the temperature for introduction into the reactor, the desired temperature may be about 50 to about 500 °C, preferably about 100 to about 300 °C. In the case in which the temperature is less than 50 °C, the catalyst becomes inactivated due to a low temperature, flowability in the reactor is deteriorated due to a high viscosity of the mixture, permeability of hydrogen to the phthalate compound and alcohol in the liquid phase state is deteriorated, such that the reaction is not suitably performed in the reactor, and in the case in which the temperature is more than 500 °C, decomposition of the reactant may be increased, it may be difficult to manufacture the reactor, and it may be difficult to control heat, or the like, due to a rapid reaction.

The mixture including the phthalate compound and alcohol of which the pressure and temperature are raised by the above-mentioned process is introduced into the reactor filled with the hydrogenation catalyst. In addition, gas-phase hydrogen (H₂) is introduced into the reactor through a separate supply line, such that the hydrogenation reaction is performed.

In this case, pressure and temperature conditions of hydrogen may be adjusted so as to be equal to the pressure and temperature conditions of the mixture including the phthalate compound and alcohol, that is, to be in a pressure range of about 50 to about 500 bar, preferably about 100 to about 300 bar and a temperature range of about 50 to about 500 °C, preferably about 100 to about 300 °C.

The hydrogenation catalyst may contain a Group 8 transition metal as an active ingredient. That is, the hydrogenation catalyst may contain, preferably, at least one selected from ruthenium (Ru), nickel (Ni), palladium (Pd), rhodium (Rh), platinum (Pt), and the like.

An aromatic ring of the phthalate compound is hydrogenated by the hydrogenation reaction as described above to thereby be converted into the cyclohexane dicarboxylate compound corresponding thereto.

The reactor is not particularly limited as long as it may be used in the art to which the present invention pertains. That is, any one of the batch type reactor or the continuous type reactor may be used. In addition, the reactor may include a heat control device controlling heat generated during the reaction.

After the reaction is terminated, the produced liquid-phase hydrogenation reaction product and unreacted gas-phase raw material are separated from each other. The separated gas-phase raw material may be recirculated in the hydrogenation process. The recovered hydrogenation reaction product may be finally separated through a decompression and cooling process.

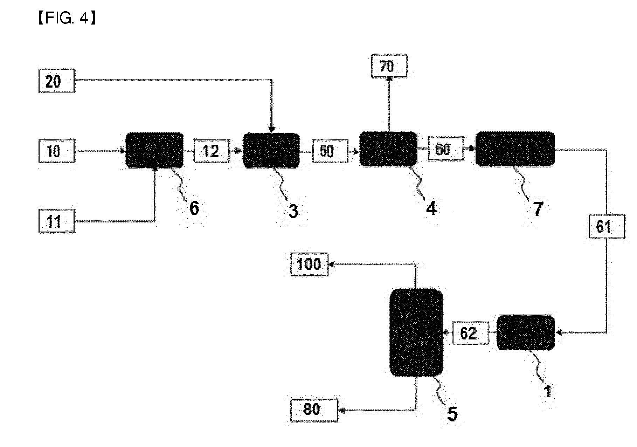

FIG. 4 is a view schematically showing a hydrogenation reaction apparatus used in a hydrogenation method according to the present invention.

Referring to FIG. 4, the hydrogenation reaction apparatus may be composed of a liquid-phase raw material mixer 6, a catalytic reactor (that is, multi-pipe type reactor) 3, a gas-liquid separator 4, a decompression device 7, a heat exchanger 1, and a separation device (reactant recovery system) 5.

Describing a process using the hydrogenation reaction apparatus in detail, first, a phthalate compound 10 and an alcohol 11 are uniformly mixed with each other in the liquid-phase raw material mixer 6. The mixed liquid-phase raw material 12 is subjected to a temperature and pressure raising process and then supplied to the catalytic reactor 3 in a suitable temperature and pressure state. Separately, hydrogen 20 is also subjected to a temperature and pressure raising process and transferred to an upper end of the catalytic reactor 3, such that a hydrogenation reaction is performed.

A reaction mixture 50 discharged from the catalytic reactor 3 is transferred to the gas-liquid separator 4, a liquid-phase reaction product 60 is transferred to the decompression device 7 by the gas-liquid separator 4, and a gas-phase unreacted material 70 is circulated in order to be discharged or recycled. A liquid-phase reaction product 61 decompressed through the decompression device 7 is cooled through the heat exchanger 1. A cooled reaction product 62 is finally subjected to an additional purification process through the separation device (reactant recovery system) 5, such that impurities 100 are removed, thereby obtaining a final reaction product 80.

However, a position of each of the devices shown in FIG. 4 may be changed, and if necessary, other devices that are not shown in FIG. 4 may be included. Therefore the hydrogenation method according to the present invention is not limited to the apparatus and the process sequence shown in FIG. 4. For example, the decompression device 14 may be positioned in front of the gas-liquid separator 13.

According to the hydrogenation method of the present invention as described above, a hydrogenation reaction conversion rate of the phthalate compound may be improved by about 10 % or more, preferably about 20% or more as compared to the case of performing the hydrogenation reaction without containing an alcohol. In addition, after the reaction proceeds, a high hydrogenation reaction conversion rate may be maintained, thereby contributing to improving the lift time of the catalyst.

Hereinafter, actions and effects of the present invention will be described in detail with reference to specific Examples of the present invention. However, the Examples are only for illustrative purposes and are not intended to limit the scope of the present invention.

### <Example>

### Example 1

A hydrogenation reaction of a phthalate compound was performed using the reaction apparatuses of FIGS. 1 and 2.

First, based on 100 parts by weight of dioctyl terephthalate (DOTP, purity: 99%), after raising a pressure of DOTP to 150 bar, a viscosity of DOTP introduced into a reactor was lowered to 4.62 cP through a heat exchanger, and then, DOTP was injected together with hydrogen into the reactor filled with a ruthenium (Ru) catalyst.

A flow rate of injected DOTP was 9.6 kg/hr, and hydrogen was injected so that a molar ratio of hydrogen to DOTP became 5 moles.

As the reactor, a single tube having the same size as that of a tube used in a multi-pipe type reactor was used, and a length of a portion of the tube filled with the catalyst was a total of 1.5 m. In addition, the hydrogenation reaction was performed while maintaining a temperature by controlling heat generated in the reactor using hot oil.

The catalyst used in the reactor was the ruthenium (Ru) catalyst, and the reactor having a cylinder shape, a diameter of 3.2 mm, and a height of 3 mm was used.

### Example 2

A hydrogenation reaction was performed in the same manner as in Example 1 except that a viscosity of DOTP introduced in the reactor in Example 1 was 3.55 cP.

### Example 3

A hydrogenation reaction was performed in the same manner as in Example 1 except that a viscosity of DOTP introduced in the reactor in Example 1 was 2.82 cP.

### Comparative Example 1

A hydrogenation reaction was performed in the same manner as in Example 1 except that a viscosity of DOTP introduced in the reactor in Example 1 was 32.6 cP.

### Comparative Example 2

A hydrogenation reaction was performed in the same manner as in Example 1 except that a viscosity of DOTP introduced in the reactor in Example 1 was 14.9 cP.

Reaction conditions in Examples 1 to 3 and Comparative Examples 1 and 2 were shown in the following Table 1.

**[Table 1]**

| | Reactor Type | DOTP Viscosity (cP) in Reactor |
|---|---|---|
| Example 1 | Continuous Type | 4.62 |
| Example 2 | Continuous Type | 3.55 |
| Example 3 | Continuous Type | 2.82 |
| Comparative Example 1 | Continuous Type | 32.6 |
| Comparative Example 2 | Continuous Type | 14.9 |

### <Experimental Example 1>

### Evaluation of Catalytic performance

Catalytic performance in Examples 1 to 3 and Comparative Examples 1 and 2 was evaluated by the following method.

Initial Performance: After performing the hydrogenation reaction using a catalyst that was never used, a rate (%) of parts by weight of dioctyl terephthalate converted into di(2-ethylhexyl)cyclohexane-1,4-dicarboxylate (DEHCH) based on 100 parts by weight of the injected dioctyl terephthalate.

An initial reaction conversion rate and a conversion rate after continuously operating the reactor for 20 hours were calculated by performing each of the experiments using the same catalyst and the reactor having the same length.

**[Table 2]**

| | DOTP Viscosity (cP) Injected into Reactor | Initial Reaction Conversion rate (%) in Reactor | Conversion Rate (%) after 20 hr of Reaction |
|---|---|---|---|
| Example 1 | 4.62 | 56.5 | 51.1 |
| Example 2 | 3.55 | 68.4 | 63.5 |
| Example 3 | 2.82 | 77.2 | 73.2 |
| Comparative Example 1 | 32.6 | 5.3 | 1.5 |
| Comparative Example 2 | 14.9 | 7.4 | 2.2 |

Referring to the results shown in Table 2, it may be confirmed that in the case in which the viscosity of the reaction raw material was high as in Comparative Examples 1 and 2, the conversion rate was significantly different even in the same reaction system, and catalytic performance depending on an operation time was also rapidly decreased. It may be appreciated that in the case of the liquid-phase raw material having a high viscosity as described above, a thick film was formed on a catalyst surface, such that the hydrogenation reaction by the catalyst was not suitably performed, and efficiency of the catalyst was rapidly decreased due to regions of the catalyst at which the high viscosity material itself was physically adsorbed or retained.

On the other hand, in Examples 1 to 3 according to the present invention, the viscosity of the reaction raw material injected into the reactor was low, such that the conversion rate was still excellent after 20 hours of the reaction. That is, it may be appreciated that in performing the hydrogenation reaction on the liquid-phase raw material over the catalyst through the cylindrical multi-pipe type reactor, the reaction should be performed in a state in which the viscosity in the reactor was 10 cP or less by adjusting the reaction temperature and pressure with respect to a material of which the liquid-phase raw material was 20 cP or more at room temperature.

### Example 4

Based on 100 parts by weight of dioctyl terephthalate (DOTP, purity: 99%), 20 parts by weight of octanol was mixed with DOTP and injected into a catalytic reactor through a pump. After DOTP was pre-heated at a pressure of 150 bar and a temperature of 120°C, and hydrogen (H₂) was also pre-heated at the same pressure and temperature, the preheated DOTP and hydrogen were supplied to an upper end of the reactor, respectively.

The reactor was a single tube, and a hydrogenation reaction was performed while controlling heat generated in the reactor through hot oil in an external jacket to maintain the temperature.

The catalyst used in the reactor was the ruthenium (Ru) catalyst, and the reactor having a cylinder shape, a diameter of 3.2 mm, and a height of 3 mm was used.

### Example 5

A hydrogenation reaction was performed in the same manner as in Example 4 except for mixing 20 parts by weight of n-butanol instead of octanol.

### Example 6

A hydrogenation reaction was performed in the same manner as in Example 4 except for mixing 20 parts by weight of ethanol instead of octanol.

### Example 7

Based on 100 parts by weight of dioctyl terephthalate (DOTP), 20 parts by weight of octanol was mixed with DOTP and filled in a batch type catalytic reactor. Thereafter, hydrogen was continuously supplied at 1 normal liter per minute (NLPM). At this time, the mixing was continuously performed using an internal stirrer in the reactor, and a reaction pressure and temperature were maintained to 150 bar and 140 °C, respectively.

The reactor was a single tube, and a hydrogenation reaction was performed while controlling heat generated in the reactor through hot oil in an external jacket to maintain the temperature.

The catalyst used in the reactor was the ruthenium (Ru) catalyst, and the reactor having a cylinder shape, a diameter of 3.2 mm, and a height of 3 mm was used.

### Comparative Example 3

A hydrogenation reaction was performed in the same manner as in Example 4 except that alcohols in Example 4 were not mixed.

### Comparative Example 4

A hydrogenation reaction was performed in the same manner as in Example 4 except for mixing 20 parts by weight of methanol instead of octanol.

### Comparative Example 5

A hydrogenation reaction was performed in the same manner as in Example 7 except that alcohols in Example 7 were not mixed.

Reaction conditions in Examples 4 to 7 and Comparative Examples 3 to 5 were shown in the following Table 3.

**[Table 3]**

| | Reactor Type | Kind and Content of Alcohol |
|---|---|---|
| Example 4 | Continuous Type | Octanol (20 Parts by Weight) |
| Example 5 | Continuous Type | Butanol (20 Parts by Weight) |
| Example 6 | Continuous Type | Ethanol (20Parts by Weight) |
| Example 7 | Batch Type | Octanol (20 Parts by Weight) |
| Comparative Example 3 | Continuous Type | Not Contained |
| Comparative Example 4 | Continuous Type | Methanol (20Parts by Weight) |
| Comparative Example 5 | Batch Type | Not Contained |

### <Experimental Example 2>

### Evaluation of Catalytic Performance

Catalytic performance in Examples 4 to 7 and Comparative Examples 3 to 5 was evaluated by the following method.

Initial Performance: After performing the hydrogenation reaction using a catalyst that was never used, a rate (%) of parts by weight of dioctyl terephthalate converted into di(2-ethylhexyl)cyclohexane-1,4-dicarboxylate (DEHCH) based on 100 parts by weight of the injected dioctyl terephthalate.

Relative initial Performance: A relative value to the initial performance when the initial performance of a hydrogenation reaction performed in a state in which alcohols were not contained was set as 1 was calculated in each of Examples.

Performance after Reaction of DOTP (100 kg): After performing a hydrogenation reaction on 100 kg of DOTP, a conversion rate of DOTP converted into di(2-ethylhexyl)cyclohexane-1,4-dicarboxylate based 100 parts by weight of injected DOTP was calculated.

Relative Performance after Reaction of DOTP (100 kg): When the performance of the hydrogenation reaction after the reaction of 100 kg of DOTP in a state in which alcohols were not contained was set as 1, a relative value of performance after the reaction of 100 kg of DOTP was calculated in each of the Examples.

Life Time of Catalyst: The life time was calculated by dividing relative performance after reaction of DOTP (100 kg) into relative performance of the initial reaction.

**[Table 4]**

| | Initial Performance (Unit: %) | Relative Performance | Performance after Reaction of DOTP (100kg) (Unit: %) | Relative Performance after Reaction of DOTP (100kg) | Life Time of Catalyst |
|---|---|---|---|---|---|
| Example 4 | 44.2 | 1.300 | 30.2 | 3.545 | 2.727 |
| Example 5 | 41.5 | 1.220 | 21.80 | 2.559 | 2.097 |
| Example 6 | 43.5 | 1.279 | 30.90 | 3.627 | 2.835 |
| Comparative Example 3 | 34.0 | 1.000 | 8.52 | 1.000 | 1.000 |
| Comparative Example 4 | 5.6 | 0.165 | 2.30 | 0.270 | 1.639 |

**[Table 5]**

| | Initial Performance (Unit: %) | Relative Performance | Performance after Reaction of DOTP (100kg) (Unit: %) | Relative Performance after Reaction of DOTP (100kg) | Life Time of Catalyst |
|---|---|---|---|---|---|
| Example 7 | 54.2 | 1.215 | 48.7 | 1.330 | 1.094 |
| Comparative Example 5 | 44.6 | 1.000 | 36.6 | 1.000 | 1.000 |

Referring to Tables 4 and 5, it may be appreciated that in the cases of Examples in which the hydrogenation reaction was performed in a state in which the alcohol having at least two carbon atoms was mixed, the conversion rate was increased by at least 20% regardless of the type of the reactor as compared to the case in which the alcohol was not contained, such that performance of the catalyst was improved. Further, even in the case of comparing the conversion rate with the passage of the reaction, in the hydrogenation method according to an exemplary embodiment of the present invention, the relative conversion rate was still high as compared to Comparative Example, such that there also was an effect of increasing the life time of the catalyst.

However, in the case of Comparative Example 4 in which methanol was mixed, relative performance was decreased as compared to Comparative Example 3 in which alcohols were not mixed, such that there was no effect of improving performance of the catalyst.

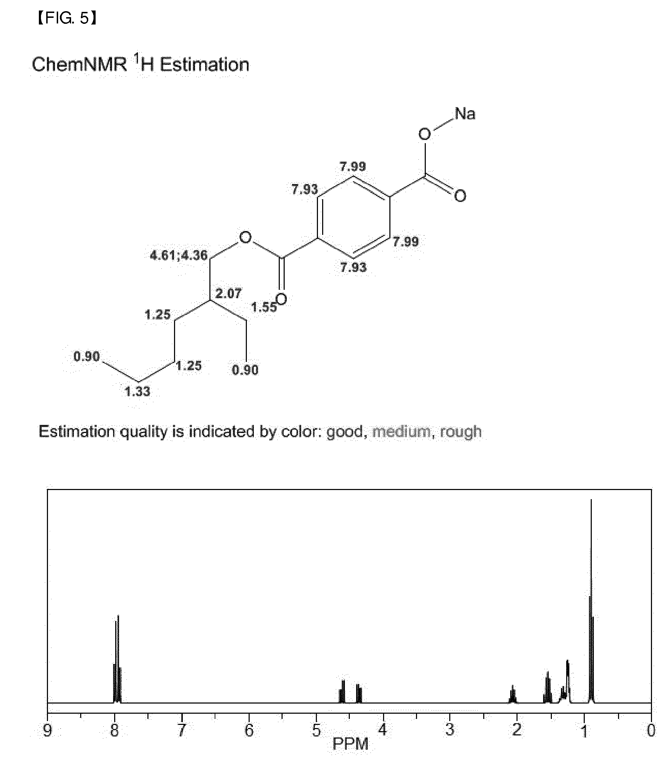

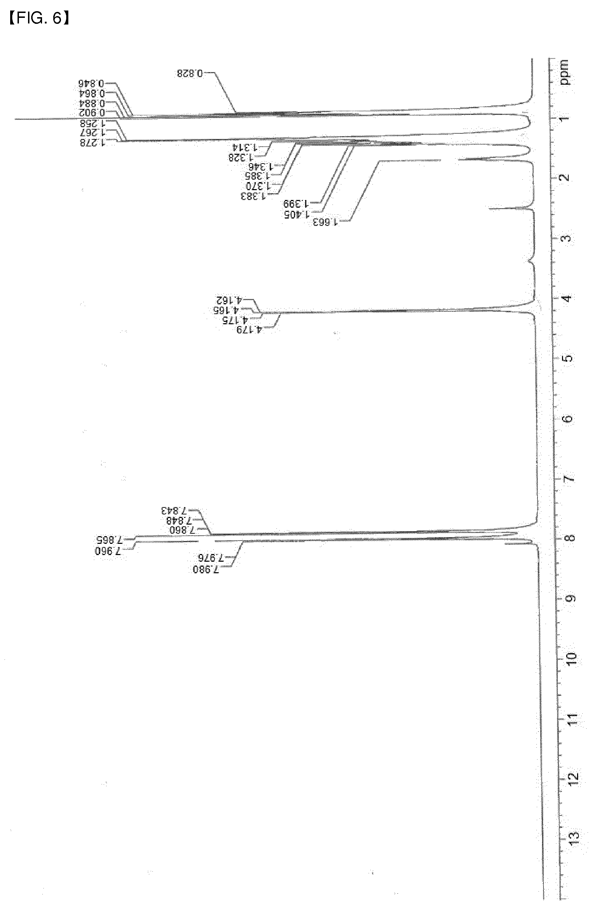

FIG. 5 shows results obtained by performing nuclear magnetic resonance (NMR) analysis on an ingredient adsorbed in a catalyst surface after the hydrogenation reaction according to Comparative Example 3, and FIG. 6 is an enlarged view of the NMR graph of FIG. 5.

Referring to FIGS. 5 and 6, it may be appreciated that a Na salt compound of terephthalic acid was adsorbed on the catalyst surface. Therefore, it may be estimated that this metal salt compound acts as a catalyst poison, thereby causing deterioration in activity of the catalyst.

However, in the ingredient recovered from the catalyst surface after the hydrogenation reaction according to the present invention, this metal salt compound was not detected at a detectable level. Therefore, it may be appreciated that the alcohol ingredient having at least two carbon atoms effectively dissolving this metal salt compound to prevent the metal salt compound from being adsorbed on the catalyst, thereby maintaining the activity and life time of the catalyst to contribute to improving performance.

### [Description of Symbols]

1: First liquid-phase raw material heat exchanger (for raising temperature)
2: Second liquid-phase raw material heat exchanger (for raising temperature)
3: Multi-pipe type reactor
4: Gas-liquid separator
5: Separation device (Reactant Recovery system)
6: Liquid-phase raw material mixer
7: Decompression device

## Claims

1. A method for hydrogenation of a phthalate compound, the hydrogenation method comprising:
lowering a viscosity in a reactor to 10 cp or less with respect to a phthalate compound having a viscosity of 20 cP or more at room temperature, and then reacting hydrogen with the phthalate compound, in the presence of a hydrogenation catalyst in the reactor.

2. The method for hydrogenation of claim 1, wherein the phthalate compound is used in the reaction in a state in which the viscosity thereof is lowered to 10 cP or less through raising a pressure and temperature using at least one heat exchanger.

3. The method for hydrogenation of claim 2, wherein the raising of the pressure and temperature is performed using at least one heat exchanger at a pressure of 50 to 500 bar and a temperature of 50 to 500 °C.

4. The method for hydrogenation of claim 1, wherein the phthalate compound corresponding to a liquid-phase raw material injected into the reactor is used in a state in which the viscosity thereof is 0.2 to 10 cP.

5. The method for hydrogenation of claim 1, wherein the phthalate compound is at least one selected from phthalate, terephthalate, isophthalate, and carboxylic acid compounds thereof.

6. The method for hydrogenation of claim 5, wherein the phthalate compound is dioctyl terephthalate (DOTP).

7. The method for hydrogenation of claim 1, wherein the hydrogenation catalyst contains at least one selected from the group consisting of ruthenium (Ru), palladium (Pd), rhodium (Rh), platinum (Pt), and nickel (Ni).

8. The method for hydrogenation of claim 1, wherein it includes:
raising a pressure and temperature of the phthalate compound in order to lower the viscosity of the phthalate compound introduced into the reactor to 10 cP or less;
supplying the phthalate compound having a viscosity of 10 cP or less and gas-phase hydrogen in the reactor filled with the catalyst; and
reacting the phthalate compound having a viscosity of 10 cP or less with hydrogen.

9. The method for hydrogenation of claim 8, wherein the phthalate compound is reacted in a liquid-phase state, and hydrogen is reacted in a gas-phase state.

10. The method for hydrogenation of claim 1 or 8, wherein the reaction is performed at a pressure of 50 to 500 bar and a temperature of 50 to 500 °C.

11. A method for hydrogenation of a phthalate compound, the hydrogenation method comprising:
reacting the phthalate compound with hydrogen, in the presence of a hydrogenation catalyst and an alcohol having at least two carbon atoms.

12. The method for hydrogenation of claim 11, wherein the phthalate compound is at least one selected from phthalate, terephthalate, isophthalate, and carboxylic acid compounds thereof.

13. The method for hydrogenation of claim 11, wherein the phthalate compound contains a metal ion or a metal salt compound as impurities.

14. The method for hydrogenation of claim 13, wherein the phthalate compound has a purity of 98% or more.

15. The method for hydrogenation of claim 11, wherein the phthalate compound is dioctyl terephthalate (DOTP).

16. The method for hydrogenation of claim 11, wherein the alcohol has 2 to 12 carbon atoms.

17. The method for hydrogenation of claim 16, wherein the alcohol is ethanol, butanol, or octanol.

18. The method for hydrogenation of claim 11, wherein the alcohol is contained at a content of 5 to 60 parts by weight based on 100 parts by weight of the phthalate compound.

19. The method for hydrogenation of claim 11, wherein the hydrogenation catalyst contains at least one selected from the group consisting of ruthenium (Ru), palladium (Pd), rhodium (Rh), platinum (Pt), and nickel (Ni).

20. The method for hydrogenation of claim 11, wherein the reaction is performed at a pressure of 50 to 500 bar and a temperature of 50 to 500 °C

21. The method for hydrogenation of claim 11, wherein the alcohol and the phthalate compound are reacted in a liquid-phase state, and hydrogen is reacted in a gas-phase state.

22. The method for hydrogenation of claim 21, wherein it includes:
mixing the phthalate compound and the alcohol with each other to form a liquid-phase mixture;
raising a pressure and temperature of the liquid-phase mixture;
supplying liquid-phase mixture subjected to the raising of the pressure and temperature and gas-phase hydrogen in a reactor; and
reacting the phthalate compound with hydrogen.
